# EUROPEAN PATENT SPECIFICATION

(11) **EP 1 974 043 B1**
(45) Date of publication and mention of the grant of the patent: **13.11.2013**
(21) Application number: 06831473.1
(22) Date of filing: 22.12.2006
(51) Int. Cl.: C12N 15/86

(54) **VECTORS**
VEKTOREN
VECTEURS

(30) Priority: 22.12.2005 GB 0526210
(43) Date of publication of application: 01.10.2008
(73) Proprietor: Oxford Biomedica (UK) Limited, Oxford 0X4 4GA (GB)
(72) Inventor: WILKES, Fraser, Oxford OX14 4RY (GB); MISKIN, James, Oxford OX4 4GA (GB); MITROPHANOUS, Kyriacos, Oxford OX4 4GA (GB); KINGSMAN, Susan, Oxford OX4 4GA (GB)
(74) Representative: O'Brien, Simon Warwick
(86) International application number: PCT/GB2006/004917
(87) International publication number: WO 2007/072056

(56) References cited:
- WO-A-03/064665
- WO-A-2005/116225
- MITROPHANOUS K A ET AL: "STABLE GENE TRANSFER TO THE NERVOUS SYSTEM USING A NON-PRIMATE LENTIVIRAL VECTOR" GENE THERAPY, MACMILLAN PRESS LTD., BASINGSTOKE, GB, vol. 6, no. 11, 1999, pages 1808-1818, XP000914884 ISSN: 0969-7128

## Description

### Field of the Invention

The present invention relates to novel vector particles and vector particle production systems.

### Background to the Invention

The success of gene therapy techniques depends largely on the ability to achieve a combination of stable chromosomal integration and high-level, regulated expression of transferred genes in a manner safe to humans. In recent years, retroviruses have been proposed as delivery vehicles for use in gene therapy. A particularly significant feature of retroviruses is their replicative strategy which includes reverse transcription of viral RNA into linear double stranded DNA and subsequent integration of this DNA into the genome of a host cell.

During the process of infection, a retrovirus initially attaches to a specific cell surface receptor. On entry into the susceptible host cell, the retroviral RNA genome is then copied to DNA by the virally encoded reverse transcriptase which is carried inside the parent virus. This DNA is transported to the host cell nucleus where it subsequently integrates into the host genome. At this stage, it is typically referred to as the provirus. The provirus is stable in the host chromosome during cell division and is transcribed like other cellular genes. The provirus encodes the proteins and packaging machinery required to make more virus, which can leave the cell by a process sometimes called "budding".

Each virus comprises genes called *gag, pol* and *env* which code for virion proteins and enzymes. In the provirus, the retroviral genome is flanked at both ends by regions called long terminal repeats (LTRs). The LTRs are responsible for proviral integration, and transcription. They also serve as enhancer-promoter sequences. In other words, the LTRs can control the expression of the viral genes. Encapsidation of the retroviral RNAs occurs by virtue of a packaging (psi) sequence located at the 5' end of the genome.

In a typical recombinant retroviral vector for use in gene therapy, at least part of one or more of the *gag*, *pol* and *env* protein coding regions may be removed from the virus. This makes the retroviral vector replication-defective. The removed portions may be replaced by a nucleotide sequence of interest (NOI) in order to generate a virus capable of integrating its genome into a host genome but wherein the modified viral genome is unable to propagate itself due to a lack of structural proteins. When integrated in the host genome, expression of the NOI may occur - resulting in, for example, a therapeutic and/or a diagnostic effect. Thus, the transfer of a NOI into a site of interest is typically achieved by: incorporating the NOI into the recombinant viral vector; packaging the modified viral vector into a virion coat; and allowing transduction of a site of interest - such as a targeted cell or a targeted cell population.

It is possible to propagate and isolate quantities of retroviral vectors (e.g. to prepare suitable titres of the retroviral vector) for subsequent transduction of, for example, a site of interest, by using a combination of a packaging or helper cell line and a recombinant vector. Typically, modem retroviral vector systems consist of viral genomes bearing cis-acting vector sequences needed for transcription, reverse-transcription, integration, translation and packaging of viral RNA into the viral particles, and (2) producer cells lines which express the trans-acting retroviral gene sequences (e.g., gag, pol and env) needed for production of virus particles. By separating the cis-and trans-acting vector sequences completely, the virus is unable to maintain replication for more than one cycle of infection. However, there are shortcomings with the current use of vector-producing cell lines.

A first issue is the generation of "live virus" (i.e., replication competent retrovirus; RCR). There are at least two different mechanisms by which this can occur. Similar or identical overlapping nucleic acid sequences present on two separate DNA molecules (i. e., vector and helper sequences) can genetically recombine. One example of such overlap is between the packaging signal of the vector and the 5' region of the gag gene. Furthermore, two separate RNA strands can serve as templates for cDNA synthesis during replication of the vector/virus, and genetic recombination can occur during DNA synthesis, leading to RCR. An additional confounding factor is the endogenous retroviral gene sequences that are present in the genome of the cells in which the virus is replicating. These retroviral gene sequences provide an additional source for generating RCR.

It is therefore very desirable to minimize the overlap between the cis-and trans-acting sequences.

A second issue is the ability to make sufficient retroviral vector particles at a suitable concentration. This has led to a range of vectors being produced which result in low efficiency packaging. While the delivery of very basic RNA constructs devoid of or lacking retroviral RNA sequences remains an attractive option, this has been difficult to achieve because of a lack of suitable vectors. It has been shown that retroviral vector particles, whether wild type or as part of a vector system, randomly package cellular RNAs and consequently deliver them to an infected/transduced cell. However the packaging efficiency of such basic RNA sequences is extremely poor.

A third issue is the propensity of mouse based packaging cell lines to package endogenous retrovirus-like elements (which can contain oncogenic gene sequences) at efficiencies close to that with which they package the desired retroviral vector. Such elements, because of their retrovirus-like structure, are transmitted to the target cell to be treated at frequencies that parallel transfer of the desired retroviral vector sequence.

Thus, a need exists for novel safe and efficient vector production for the transmission of genetic materials in a mammal.

### Summary of the Invention

We describe viral vector particles which have the ability to deliver a sequence which is devoid of or lacking viral RNA. The viral vector particle which comprises a sequence that is devoid of or lacking viral RNA may be the result of removing or eliminating the viral RNA from the sequence. In one embodiment this may be achieved by using an endogenous packaging signal binding site on gag. Alternatively, the endogenous packaging signal binding site is on pol. In this embodiment the RNA which is to be delivered will contain a cognate packaging signal. In another embodiment, a heterologous binding domain (which is heterologous to gag) located on the RNA to be delivered and a cognate binding site located on gag or pol can be used to ensure packaging of the RNA to be delivered. The heterologous sequence could be non-viral or it could be viral in which case it is preferably derived from a different virus. The vector particles of the present invention could be used to deliver therapeutic RNA - in which case functional integrase and/or reverse transcriptase is not required. These vector particles could also be used to deliver a therapeutic gene of interest - in which case pol would need to be functional. As these vector particles may have a genome which is devoid of or lacking viral RNA, they will have safety advantages over particles which contain viral RNA.

In more detail, when a wild-type virus infects a cell, it integrates into the host genome, produces protein to make a particle and then needs to fill the particle with two, RNA, genomes. There are many different species of RNA in the cell and the particle needs to identify which RNA to package. In order to do this the viral genome contains a sequence of RNA which is recognized by the particle. This RNA signal is the packaging signal. Non wild-type viral vectors which are used as delivery vectors work in the same way, except that the packaging components are supplied on separate plasmids. In the present invention by including this packaging signal onto a piece of RNA (for example, RNA comprising therapeutic RNA) we can cause the particle to package said RNA. The RNA, which is to be packaged, can be transcribed from a DNA copy.

In a particularly preferred embodiment we also alter gag-pol and replace the packaging signal with a corresponding packaging signal. In this embodiment, we can cause the particle to package the RNA with the new packaging signal. The advantage of this approach is that it is possible to package an RNA sequence which is devoid of viral sequence for example, RNAi.

An alternative approach is to rely on over expression of the RNA to be packaged. As described herein, the RNA to be packaged is over expressed in the absence of any RNA containing a packaging signal. This may result in a significant level of therapeutic RNA being packaged, and that this amount is sufficient to transduce a cell and have a biological effect.

The present invention provides novel viral constructs that comprise heterologous packaging components thereby providing for increased safety of viral production systems and allowing for the packaging of RNA comprising reduced viral RNA sequence.

In a general aspect of the present invention there is provided a retroviral vector particle production system comprising
Env;
retroviral Gag, or retroviral Gag/Pol, containing a binding domain;
and an RNA sequence to be packaged containing a retroviral packaging signal or a corresponding cognate packaging signal which is recognized by said binding domain to facilitate packaging of the RNA sequence into a retroviral vector particle, wherein said RNA sequence to be packaged lacks any other retroviral sequences including viral sequences required for reverse transcription and integration.

In one embodiment of the retroviral vector particle production system of the present invention the binding domain is endogenous to retroviral Gag or retroviral Gag/Pol, the RNA sequence to be packaged contains the retroviral packaging signal recognized by said binding domain, and said RNA sequence to be packaged lacks any other retroviral sequences.

In another embodiment of the retroviral vector particle production system of the present invention the binding domain is heterologous to retroviral Gag or retroviral Gag/Pol.

In one embodiment the heterologous binding domain is retroviral, the RNA sequence to be packaged contains the retroviral packaging signal recognized by said binding domain, and said RNA sequence to be packaged lacks any other retroviral sequences.

In another embodiment the binding domain is non-viral, the RNA sequence to be packaged contains the non-viral packaging signal recognized by said binding domain, and said RNA sequence to be packaged lacks all retroviral sequences.

In another embodiment of the retroviral vector particle production system of the present invention the heterologous binding domain contains a binding domain derived from a bacteriophage coat protein, a Rev protein, a protein of the U1 small nuclear ribonucleoprotein particle, a Nova protein, a TF111A protein, a TIS11 protein, a trp RNA-binding attenuation protein (TRAP) or a pseudouridine synthase.

In another aspect of the invention there is provided a retroviral vector particle comprising Env and retroviral Gag or retroviral Gag/Pol, wherein said retroviral Gag and retroviral Gag/Pol comprises a RNA binding domain, and wherein said retroviral vector particle further comprises a packaged RNA sequence containing a retroviral packaging signal or a corresponding cognate packaging signal which is recognized by said binding domain to facilitate packaging of the RNA sequence into a viral vector particle, wherein said packaged RNA sequence lacks any other retroviral sequences including retroviral sequences required for reverse transcription and integration.

In one embodiment of the retroviral vector particle production system or retroviral vector particle of the present invention the Gag or Gag/Pol is derived from a lentivirus.

In one embodiment of the retroviral vector particle production system or retroviral vector particle of the present invention the Gag or Gag/Pol is derived from HIV or EIAV.

In one embodiment of the retroviral vector particle production system or retroviral vector particle of the present invention the RNA sequence further comprises a nucleotide sequence of interest (NOI).

In another embodiment of the retroviral vector particle production system or retroviral vector particle of the present invention the NOI is a therapeutic RNA.

In another embodiment of the retroviral vector particle production system or retroviral vector particle of the present invention the therapeutic RNA is selected from the group of siRNA, micro-RNA, ribozyme, mRNA and tRNA.

We also describe herein a polynucleotide comprising an RNA sequence which lacks sequences required for reverse transcription and or integration and contains an RNA packaging signal which recognizes a corresponding RNA binding domain associated with gag or gag/pol to facilitate packaging of the RNA sequence into a viral vector particle. Preferably, the RNA comprises further nucleotide sequences of interest such as therapeutic RNA. Preferably the therapeutic RNA is a siRNA, a micro-RNA, a ribozyme, an mRNA or a tRNA.. Preferably, the polynucleotide is used in a viral vector particle production system according to the present invention.

Preferably, the RNA packaging signal is a heterologous packaging signal.and/or the RNA binding domain is a heterologous binding domain.

We also describe herein a polynucleotide comprising a nucleotide sequence encoding a viral gag protein or retroviral gag and pol proteins wherein the gag protein or pol protein comprises a heterologous RNA binding domain capable of recognising a corresponding sequence in an RNA sequence to facilitate packaging of the RNA sequence into a viral vector particle.

Preferably the heterologous RNA binding domain comprises an RNA binding domain derived from a bacteriophage coat protein, a Rev protein, a protein of the U1 small nuclear ribonucleoprotein particle, a Nova protein, a TF111A protein, a TIS11 protein, a trp RNA-binding attenuation protein (TRAP) or a pseudouridine synthase.

Preferably the bacteriophage coat protein is *E*. *coli* phage MS2 coat protein.

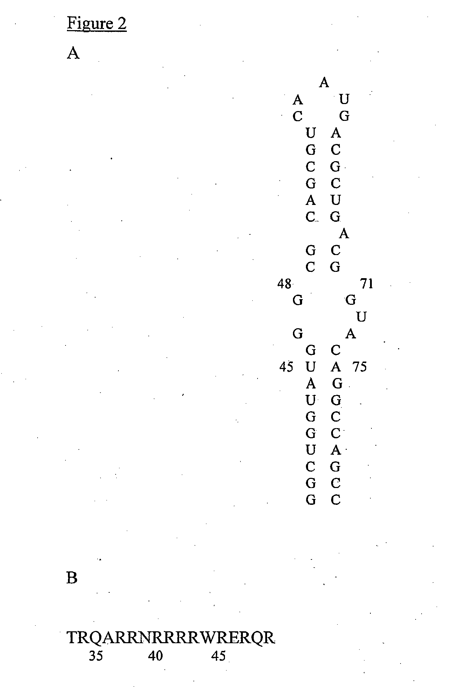

Preferably the RNA binding domain derived from Rev comprises the arginine-rich RNA binding domain, for example the sequence shown in Figure 2B or a derivative or homologue thereof.

Preferably the RNA binding domain is derived from U1A protein of the U1 small nuclear ribonucleoprotein particle.

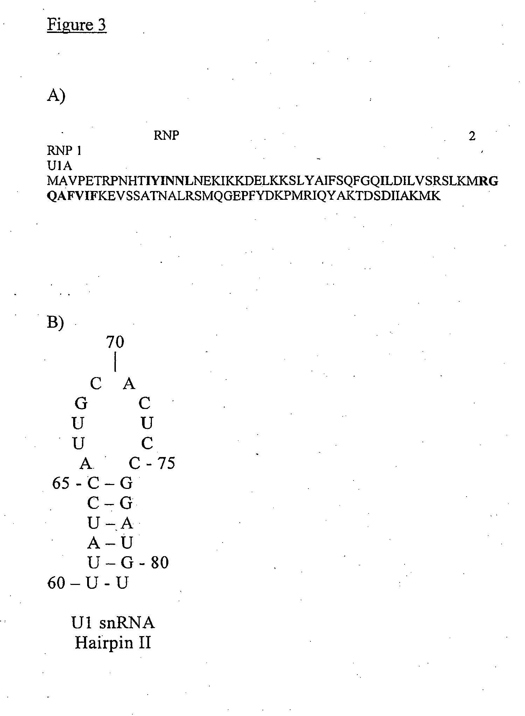

Preferably the RNA binding domain of U1A comprises the sequence shown in Figure 3A, or a derivative or homologue thereof.

Preferably the RNA binding domain is derived from a Nova protein and comprises one or more K homology (KH) motifs.

Preferably the RNA binding domain comprises zinc fingers 4 to 7 of a TFIIIA protein.

We describe that the RNA binding domain may comprise zinc fingers 4 to 6 of a TFIIIA protein.

Preferably the TIS11 protein is TIS11d.

Preferably the RNA binding domain comprises a TIS11 TZF domain.

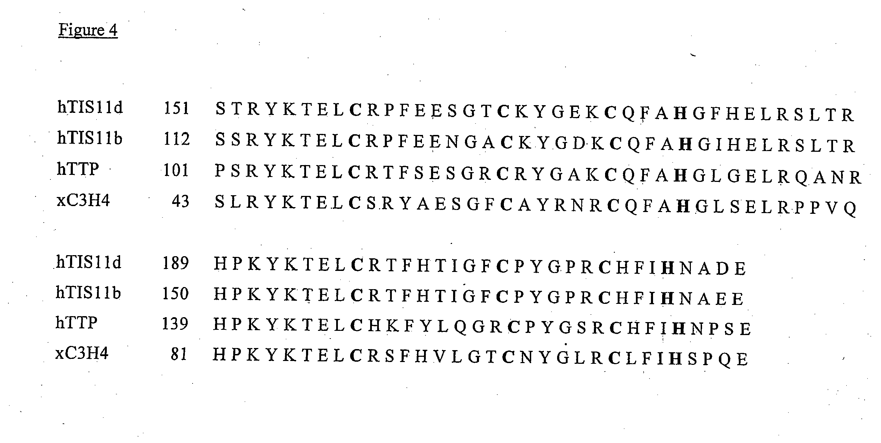

Preferably the RNA binding domain comprises the TIS11d TZF sequence shown in Figure 4, or a derivative or homologue thereof.

Preferably the pseudouridine synthase is TruB.

We describe a polynucleotide wherein the heterologous RNA binding domain is not derived from a virus.

We also describe a polynucleotide wherein the gag protein does not comprise its wild-type RNA binding domain.

We also describe a polynucleotide wherein the heterologous RNA binding domain replaces the wild-type gag RNA binding domain.

We also describe a polynucleotide wherein the heterologous RNA binding domain is positioned substantially at the end of the gag protein.

We also describe a polynucleotide wherein the gag protein is a retroviral or a lentiviral gag protein.

We also describe a polynucleotide wherein the lentiviral gag protein is derived from HIV.

We also describe a polynucleotide wherein the lentiviral gag protein is derived from a non-primate lentivirus such as EIAV.

We also describe that the polynucleotide may further comprise a nucleotide sequence encoding pol protein.

We also describe that the polynucleotide may further comprise a nucleotide sequence encoding env protein.

We also describe a polynucleotide wherein the env protein includes an RNA binding site which recognizes a site on the packageable RNA and thus packaging RNA into vesicles - this would occur in the absence of gag/pol.

We also describe herein a vector comprising a polynucleotide described herein.

We also describe herein a host cell comprising a polynucleotide described herein.

The host cell may be a viral packaging cell.

We also describe herein a viral vector comprising a heterologous packaging sequence comprising an RNA sequence capable of binding to an RNA binding domain as defined herein.

We describe that the heterologous packaging sequence may comprise an RNA hairpin located at the 5' end of the phage replicase gene.

We describe that the heterologous packaging sequence may comprise the rev RRE IIB sequence.

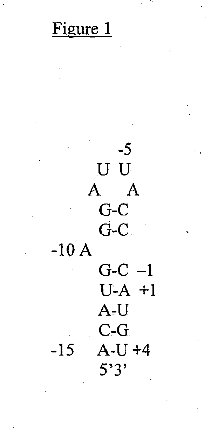

We describe that the heterologous packaging sequence may comprise the RNA hairpin shown in Figure 1.

We describe that the heterologous packaging sequence may comprise the hairpin II of U1 snRNA.

We describe that the heterologous packaging sequence may comprise the Nova binding stem loop having the sequence [(UCAU(N)₀₋₄]₃.

We describe that the heterologous packaging sequence may comprise the TIS11 binding sequence 5-UUAUUUAUU-3.

We describe that the heterologous packaging sequence comprises the TRAP binding site having eleven trinucleotide repeats, wherein the trinucleotide repeats are selected from GAG or UAG.

Preferably the heterologous packaging sequence replaces the wild-type viral packaging sequence.

Preferably the heterologous packaging sequence is positioned between the primer binding site and the polypurine tract.

Preferably the viral vector further comprises a nucleotide sequence of interest (NOI) which may encode for a therapeutic protein or for example be in the form of an antisense.

We describe that the nucleotide sequence of interest is a therapeutic RNA such as an siRNA, shRNA, a micro-RNA, or regulated micro or shRNA molecules a ribozyme, an mRNA or a tRNA.

We also describe that the viral vector may comprise no viral sequences.

We also describe that the viral vector may be derived from a retrovirus or a lentivirus.

We also describe that the lentiviral vector may be derived from HIV or EIAV.

We also describe herein a polynucleotide which is, or comprises, a therapeutic RNA associated with a packaging sequence which enables the therapeutic RNA to be packaged into a viral particle.

We also describe herein a viral vector particle production system comprising a nucleic acid sequence encoding a viral vector comprising RNA sequences containing the packaging signal recognized by gag but where in said RNA lacks sequences required for reverse transcription and or integration and a polynucleotide comprising a nucleotide sequence encoding (i) a viral gag protein or (ii) viral gag and pol proteins, wherein the gag and/or pol protein comprises the RNA binding domain capable of recognising a corresponding sequence in said RNA sequence to facilitate packaging of the RNA sequence into a viral vector particle.

We also describe herein a viral vector particle production system comprising a set of nucleic acid sequences encoding a viral vector as described herein and a polynucleotide as described herein encoding a gag protein. Accordingly, the viral vector will comprise a heterologous packaging sequence which binds to a corresponding RNA domain encoded by the gag protein.

We also describe herein a viral vector particle production system comprising a polynucleotide which comprises a nucleotide sequence encoding a viral gag protein or viral gag and pol proteins and a construct which over expresses a NOI.

Preferably the retroviral vector particle production system comprises three DNA constructs which encode (i) the viral RNA genome, (ii) gag and pol proteins, and (iii) env protein or a substitute therefore, respectively.

We also describe herein a viral vector particle production system of the present invention in a host cell.

We also describe herein a set of DNA constructs for use in the system of the present invention comprising a DNA construct comprising a polynucleotide as described herein, a DNA construct encoding a viral vector as described herein, and a DNA construct encoding env protein or a substitute therefore.

We describe that the set of DNA constructs may be in a host cell, so forming a producer cell.

According to another aspect of the present invention there is provided a viral particle produced by the system of the present invention.

We also describe herein the use of a viral vector particle of the present invention for gene therapy.

We also describe herein a viral vector particle comprising gag or gag/pol ,wherein said gag and gag/pol comprises an RNA binding domain, and wherein said vector particle further comprises an RNA sequence containing the packaging signal recognized by said RNA binding domain to facilitate packaging of the RNA sequence into a viral vector particle but where in said RNA sequence lacks sequences required for reverse transcription and or integration.

We also describe herein a viral vector particle comprising gag or gag/pol, wherein the gag and/or pol protein comprises a heterologous RNA binding domain capable of recognising a corresponding sequence in an RNA sequence to facilitate packaging of the RNA sequence into a viral vector particle and an RNA genome comprising said corresponding packaging sequence capable of recognising said heterologous RNA binding domain.

Preferably the RNA sequence of the vector particle of the invention further comprises a nucleotide sequence of interest (NOI. Preferably, the NOI is selected from the group of siRNA, a micro-RNA, a ribozyme, an mRNA and a tRNA.

### Description of Figures

Figure 1 shows an RNA hairpin which interacts with phage MS2 coat protein. The RNA hairpin consists of a 7-bp stem closed by a 4-nt loop, the stem being interrupted by an unpaired nucleotide between bp 5 and 6.
Figure 2A shows a sequence from stem-loop IIB of wild-type RRE.
Figure 2B shows amino acids 34 through 50 of the Rev RNA binding domain.
Figure 3A shows a fragment containing the first 98 residues of U1A protein which retains the full RNA-binding properties of the full length protein.
Figure 3B shows the sequence of hairpin II of U1
Figure 4 shows the mRNA binding domain of TIS11 proteins. The sequences show the highly conserved TZF domain which comprise two CCCH zinc fingers.

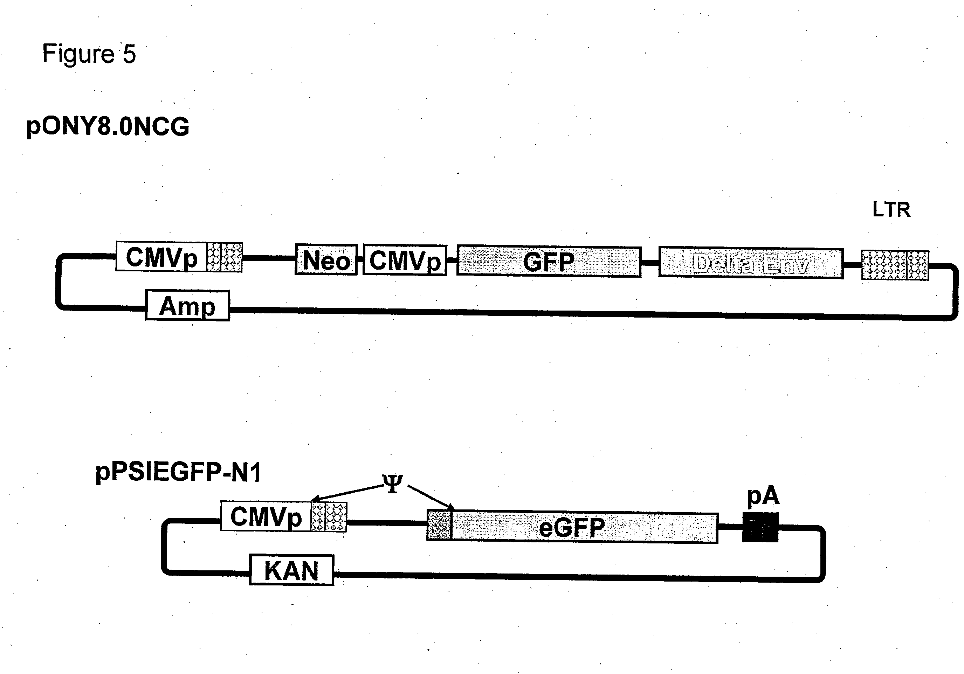

Figure 5 shows a graphic representation of the pONY8.0NCG and pPSIEGFP-N1 constructs.

### Detailed description of the invention

Various preferred features and embodiment of the present invention will now be described by way of non-limiting example.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, IRL Press; and, D. M. J. Lilley and J. E. Dahlberg, 1992, Methods of Enzymology: DNA Structure Part A: Synthesis and Physical Analysis of DNA Methods in Enzymology, Academic Press.

It is desirable, in a therapeutic setting, to be able to transiently express proteins or transiently knock down expression of proteins. One way of doing this would be to deliver RNA to target cells. RNA has a finite lifetime in cells and once it has been degraded the phenotype it conferred on cells would be removed. This has been difficult to achieve to date because of a lack of suitable vectors. Transfection methods for RNA have yet to achieve satisfactory levels of transfer *in vivo.*

We propose to use retroviral or lentiviral packaging components to package therapeutic RNA for efficient delivery *in vivo.*

It has been shown that retroviral or lentiviral particles, whether wild type or as part of a vector system, randomly package cellular RNAs, and consequently deliver them to an infected/ transduced cell.

The system we propose here would be capable of being free of retroviral or lentiviral vector RNA sequence. This may be achieved by including an RNA binding protein in gag or gag/pol (the gag and gag/pol proteins make up the viral particle). This RNA binding protein would be capable of binding a specific (or cognate) RNA sequence and this cognate sequence could be inserted into the RNA entity that is to be delivered to the target cells.

An alternative approach is to rely on overexpression of the RNA to be packaged. Thus the RNA to be packaged is overexpressed in the absence of any RNA containing a packaging signal. This may result in a significant level of therapeutic RNA being packaged, and this amount would be sufficient to infect a cell and have a biological effect.

To make the vector a transient transfection may be carried out with the modified gag or gag/pol construct plus an envelope expression construct and the plasmid which allows the generation of the transcript (now containing the RNA sequence to which the RNA binding protein binds) to be delivered to the target cells. The RNA transcript would be sequestered into the viral particle (by virtue of the interaction between the RNA binding protein and its cognate RNA sequence) which would bud from the cells and would contain the envelope protein on its surface. The vector can now be used to deliver RNA to target cells. The envelope protein would bind to the receptors on the target cells and deliver the RNA transcript to the intracellular environment where it can either be processed (e.g. by splicing) or translated into protein or function at the RNA level (e.g. RNAi, antisense, etc).

The viral particle is made using gag or gag/pol in their entirety or modified. If pol is absent, reverse transcription or integration would not occur. Other RNA binding proteins and RNA sequences can be used or new ones can be generated or evolved.

### Polynucleotides

Polynucleotides of the invention may comprise DNA or RNA. They may be single-stranded or double-stranded. It will be understood by a skilled person that numerous different polynucleotides can encode the same polypeptide as a result of the degeneracy of the genetic code. In addition, it is to be understood that skilled persons may, by using routine techniques, make nucleotide substitutions that do not affect the polypeptide sequence encoded by the polynucleotides used in the invention to reflect the codon usage of any particular host organism in which the polypeptides are to be expressed. The polynucleotides may be modified by any method available in the art. Such modifications may be carried out in order to enhance the *in vivo* activity or life span of polynucleotides of the invention.

Polynucleotides such as DNA polynucleotides may be produced recombinantly, synthetically, or by any means available to those of skill in the art. They may also be cloned by standard techniques.

Longer polynucleotides will generally be produced using recombinant means, for example using PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

It will be appreciated that the polynucleotide of the invention may contain only coding regions. However, it is preferred if the polynucleotide further comprises, in operable linkage, a portion of nucleic acid that allows for efficient translation of the coding sequence. It is further preferred that the polynucleotide (when in a DNA form) further comprises a promoter in operable linkage which allows for the transcription of the coding region and the portion of nucleic acid that allows for efficient translation of the coding region in a target cell.

### Protein

As used herein, the term "protein" includes single-chain polypeptide molecules as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulfide bonds. The terms subunit and domain may also refer to polypeptides and peptides having biological function. For example, the heterologous RNA binding domain of the present invention may comprise a full length heterologous protein, or a fragment thereof which retains RNA binding properties.

### Derivatives

The term "derived from" is used in its normal sense as meaning the sequence need not necessarily be obtained from a sequence but instead could be derived therefrom. By way of example, a sequence may be prepared synthetically or by use of recombinant DNA techniques.

### Viruses

As it is well known in the art, a vector is a tool that allows or facilitates the transfer of an entity from one environment to another. In accordance with the present invention, and by way of example, some vectors used in recombinant DNA techniques allow entities, such as a segment of DNA (such as a heterologous DNA segment, such as a heterologous cDNA segment), to be transferred into a host cell for the purpose of replicating the vectors comprising a segment of DNA. Examples of vectors used in recombinant DNA techniques include but are not limited to plasmids, chromosomes, artificial chromosomes or viruses.

The retroviral vector of the present invention may be derived from or may be derivable from any suitable retrovirus. A large number of different retroviruses have been identified. Examples include: murine leukemia virus (MLV), human T-cell leukemia virus (HTLV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV). A detailed list of retroviruses may be found in Coffin et al., 1997, "retroviruses", Cold Spring Harbour Laboratory Press Eds: JM Coffin, SM Hughes, HE Varmus pp 758-763.

Retroviruses may be broadly divided into two categories: namely, "simple" and "complex". Retroviruses may even be further divided into seven groups. Five of these groups represent retroviruses with oncogenic potential. The remaining two groups are the lentiviruses and the spumaviruses. A review of these retroviruses is presented in Coffin *et al.,* 1997 (*ibid*).

The basic structure of retrovirus and lentivirus genomes share many common features such as a 5' LTR and a 3' LTR, between or within which are located a packaging signal to enable the genome to be packaged, a primer binding site, integration sites to enable integration into a host cell genome and *gag, pol* and *env* genes encoding the packaging components - these are polypeptides required for the assembly of viral particles. Lentiviruses have additional features, such as *rev* and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration, and transcription. LTRs also serve as enhancer-promoter sequences and can control the expression of the viral genes.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

In a defective retroviral vector genome *gag, pol* and *env* may be absent or not functional. The R regions at both ends of the RNA are repeated sequences. U5 and U3 represent unique sequences at the 5' and 3' ends of the RNA genome respectively.

In a typical retroviral vector of the present invention, at least part of one or more protein coding regions essential for replication may be removed from the virus. This makes the viral vector replication-defective. Portions of the viral genome may also be replaced by a library encoding candidate modulating moieties operably linked to a regulatory control region and a reporter moiety in the vector genome in order to generate a vector comprising candidate modulating moieties which is capable of transducing a target non-dividing host cell and/or integrating its genome into a host genome.

Lentivirus vectors are part of a larger group of retroviral vectors. A detailed list of lentiviruses may be found in Coffin et al ("Retroviruses" 1997 Cold Spring Harbour Laboratory Press Eds: JM Coffin, SM Hughes, HE Varmus pp 758-763). In brief, lentiviruses can be divided into primate and non-primate groups. Examples of primate lentiviruses include but are not limited to: the human immunodeficiency virus (HIV), the causative agent of human auto-immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

The lentivirus family differs from retroviruses in that lentiviruses have the capability to infect both dividing and non-dividing cells (Lewis *et al*1992; Lewis and Emerman 1994). In contrast, other retroviruses - such as MLV - are unable to infect non-dividing or slowly dividing cells such as those that make up, for example, muscle, brain, lung and liver tissue.

A lentiviral or lentivirus vector, as used herein, is a vector which comprises at least one component part derivable from a lentivirus. Preferably, that component part is involved in the biological mechanisms by which the vector infects cells, expresses genes or is replicated.

The lentiviral vector may be a "non-primate" vector, i.e., derived from a virus which does not primarily infect primates, especially humans.

The examples of non-primate lentivirus may be any member of the family of lentiviridae which does not naturally infect a primate and may include a feline immunodeficiency virus (FIV), a bovine immunodeficiency virus (BIV), a caprine arthritis encephalitis virus (CAEV), a Maedi visna virus (MVV) or an equine infectious anaemia virus (EIAV).

In one embodiment the viral vector is derived from EIAV. EIAV has the simplest genomic structure of the lentiviruses and is particularly preferred for use in the present invention. In addition to the *gag, pol* and *env* genes EIAV encodes three other genes: *tat*, *rev*, and *S2*. *Tat* acts as a transcriptional activator of the viral LTR (Derse and Newbold, 1993; Maury et al., 1994) and Rev regulates and coordinates the expression of viral genes through rev-response elements (RRE) (Martarano et al 1994). The mechanisms of action of these two proteins are thought to be broadly similar to the analogous mechanisms in the primate viruses (Martano et al ibid). The function of S2 is unknown. In addition, an EIAV protein, Ttm, has been identified that is encoded by the first exon of *tat* spliced to the *env* coding sequence at the start of the transmembrane protein.

Preferred vectors of the present invention are recombinant retroviral or lentiviral vectors.

The term "recombinant retroviral or lentiviral vector" (RRV) refers to a vector with sufficient retroviral genetic information to allow packaging of an RNA genome, in the presence of packaging components, into a viral particle capable of infecting a target cell. Infection of the target cell may include reverse transcription and integration into the target cell genome. The RRV carries non-viral coding sequences which are to be delivered by the vector to the target cell. A RRV is incapable of independent replication to produce infectious retroviral particles within the final target cell. Usually the RRV lacks a functional *gag-pol* and/or *env* gene and/or other genes essential for replication. The vector of the present invention may be configured as a split-intron vector. A split intron vector is described in PCT patent application WO 99/15683.

Preferably the RRV vector of the present invention has a minimal viral genome.

As used herein, the term "minimal viral genome" means that the viral vector has been manipulated so as to remove the non-essential elements and to retain the essential elements in order to provide the required functionality to infect, transduce and deliver a nucleotide sequence of interest to a target host cell. Further details of this strategy can be found in our WO98/17815.

A minimal viral genome of the present invention may comprise (5') R - U5 - one or more nucleotide sequence of interest sequences - U3-R (3').

In one embodiment, the minimal viral genome comprises no viral sequences. For example, it may only comprise a NOI (e.g., an siRNA) and a packaging signal.

However, the plasmid vector used to produce the viral genome within a host cell/packaging cell will also include transcriptional regulatory control sequences operably linked to the retroviral genome to direct transcription of the genome in a host cell/packaging cell. These regulatory sequences may be the natural sequences associated with the transcribed retroviral sequence, i.e. the 5' U3 region, or they may be a heterologous promoter such as another viral promoter, for example the CMV promoter. Some lentiviral genomes require additional sequences for efficient virus production. For example, in the case of HIV, *rev* and RRE sequence are preferably included. However the requirement for *rev* and RRE may be reduced or eliminated by codon optimization. Further details of this strategy can be found in our WO01/79518. Alternative sequences which perform the same function as the rev/RRE system are also known. For example, a functional analogue of the rev/RRE system is found in the Mason Pfizer monkey virus. This is known as the constitutive transport element (CTE) and comprises an RRE-type sequence in the genome which is believed to interact with a factor in the infected cell. The cellular factor can be thought of as a *rev* analogue. Thus, CTE may be used as an alternative to the rev/RRE system. Any other functional equivalents which are known or become available may be relevant to the invention. For example, it is also known that the Rex protein of HTLV-I can functionally replace the Rev protein of HIV-1. It is also known that Rev and Rex have similar effects to IRE-BP.

### Packaging Sequence

As utilized within the context of the present invention the term "packaging signal" which is referred to interchangeably as "packaging sequence" or "psi" is used in reference to the non-coding, cis-acting sequence required for encapsidation of retroviral RNA strands during viral particle formation. In HIV-1, this sequence has been mapped to loci extending from upstream of the major splice donor site (SD) to at least the gag start codon.

As used herein, the term "extended packaging signal" or "extended packaging sequence" refers to the use of sequences around the psi sequence with further extension into the gag gene. The inclusion of these additional packaging sequences may increase the efficiency of insertion of vector RNA into viral particles. As an example, for the Murine Leukemia Virus MoMLV, the minimum core packaging signal is encoded by the sequence (counting from the 5' LTR cap site) from approximately nucleotide 144, up through the Pst I site (approx. nucleotide 567). The extended packaging signal of MoMLV includes the sequence beyond nucleotide 567 up through the start of the gag/pol gene (approx. nucleotide 621), and beyond nucleotide 1040 (Bender et al., 1987). These sequences include about a third of the gag gene sequence.

Feline immunodeficiency virus (FIV) RNA encapsidation determinants have been shown to be discrete and non-continuous, comprising one region at the 5' end of the genomic mRNA (R-U5) and another region that mapped within the proximal 311 nt of gag. (Kaye et al., 1995) showed that mRNAs of subgenomic vectors as well as of full-length molecular clones were optimally packaged into viral particles and resulted in high-titer FIV vectors when they contained only the proximal 230 nucleotides (nt) of gag. Further 3' truncations of gag sequences progressively diminished encapsidation and transduction. Deletion of the initial ninety 5' nt of the gag gene abolished mRNA packaging, demonstrating that this segment is indispensable for encapsidation.

As defined herein, by "heterologous packaging sequence" it is meant a packaging sequence that does not originate from the native retroviral vector.

The heterologous packaging sequence may replace none, all or part of the original packaging signal of the viral genome using standard molecular biology techniques. In one embodiment, the heterologous packaging sequence is positioned between the primer binding site and the polypurine tract.

In conjunction, the gag or gag-pol protein (or polynucleotide encoding therefore) is modified to comprise an RNA binding domain which binds to the heterologous packaging signal. This RNA binding domain may be positioned in the nucleocapsid protein which codes for the packaging recognition amino acids. In one embodiment, the wild-type RNA binding amino acids of gag are removed and replaced with the heterologous RNA binding domain. This is achieved using standard molecular biology techniques. In another embodiment the heterologous RNA binding protein or domain is positioned elsewhere, for example at the end of, or substantially at the end of either gag or gag-pol, or inserted within the dUTPase domain of the pol protein.

### Examples of Heterologous Packaging Systems

The present invention makes use of heterologous RNA binding proteins and their corresponding RNA target sequences. The invention relates also to RNA binding domains derived from RNA binding proteins, whereby the binding proteins retain their ability to recognize the corresponding RNA target sequences. Examples of RNA binding proteins, RNA binding domains and their corresponding sequences are detailed below. However, it will be appreciated that these examples are not limiting and that the use of any suitable RNA binding protein and its corresponding RNA target sequence is encompassed by the present invention.

### bacteriophage coat protein

The coat protein of the *Escherichia coli* phage MS2 interacts specifically with a 19-nt-long RNA hairpin located at the 5' end of the viral replicase gene (Grahn et al., 2001). This RNA hairpin consists of a 7-bp stem closed by a 4-nt loop, the stem being interrupted by an unpaired nucleotide between bp 5 and 6 (Fig. 1).

The RNA hairpin binds to a dimer of the MS2 coat protein. Binding assays with natural sequence variants have shown that for tight binding of the hairpin to a dimer of the coat protein it is important to maintain the base-paired stem and to have a purine at the unpaired - 10 position. In the loop, strong binding also requires a pyrimidine at position -5 and adenines at both positions -7 and -4 (Witherell et al., 1991).

In the wild-type hairpin there is a uracil at position -5, which, when substituted by a cytosine, has been shown to bind 5-50 times stronger to the coat protein (Lowary & Uhlenbeck, 1987; Talbot et al., 1990; Lago et al., 1998). Substitution by either purine, however, results in binding 10-100 times weaker than the wild-type (Carey et al., 1983a).

### Rev Protein

The retroviral Rev protein is an RNA-binding protein that regulates viral gene expression by affecting the relative amounts of spliced and unspliced mRNAs that are exported to the cytoplasm (Battiste et al., 1996). Rev mediates its function by binding to the RRE RNA located within the *env* gene (Olsen et al., 1990; Zapp et al., 1991). A stem-loop structure known as RRE IIB has been identified as the high affinity Rev-binding site and contains an asymmetric internal bulge and at least one non Watson Crick base pair element (Fig 2A) (Tan et al., 1993). Short α-helical peptides corresponding to the arginine-rich RNA-binding domain of Rev (Fig. 2B) have been shown to bind to this small RNA element with a specificity similar to the intact protein (Tan et al., Cell 73, 1031 (1993).

### U1 Small Nuclear Ribonucleoprotein Particle

The U1A protein is a component of the U1 small nuclear ribonucleoprotein particle (U1 snRNP), which is one of five large RNA-protein complexes involved in pre-mRNA splicing (Lührmann et al., 1990; Nagai et al., 1994). U1A protein contains two copies of RNP motif separated by a protease-sensitive linker (Sillekens et al, 1987). This motif functions as an RNA-binding module in over 150 distinct proteins involved in RNA processing and transport (Burd et al., 1994; Birney et al., 1993). The RNP motif is characterized by two short highly conserved sequences, called RNP1 and RNP2, embedded within a more weakly conserved sequence of∼80 amino acid residues. Proteins containing the RNP motif are known to bind various forms of RNA, including single-strands, hairpins and internal loops. U1A protein binds to hairpin II of U1 snRNA (Scherley et al., 1989; Bach et al., 1990) (Fig. 3B), and a fragment containing the first 98 residues retains the full RNA-binding properties of the whole protein (Fig. 3A) (Scherley et al., 1990; Hall, 1994).

RNA binding studies of various point mutants of U1A protein showed that residues within the RNP1 and RNP2 motifs are likely to be involved in RNA binding, and that basic residues on the loops between the β1 strand and helix (A) and between the β2 and β3 strands also make important interactions (Nagai et al, 1990; Jensen et al., 1991).

It has been found that U1A protein binds to the 3'-untranslated region (3'UTR) of its own pre-mRNA (Boelens et al., 1993). Two molecules of U1A protein bind to a region upstream of the polyadenylation signal and inhibit cleavage and polyadenylation of the pre-mRNA, thus autoregulating its expression.

### Nova Protein

The Nova family of proteins were identified as target antigens in the paraneoplastic opsoclonus-myoclonus ataxia (POMA) syndrome, a disorder in which motor dysfunction suggests a defect in the inhibitory control of neurons in the brainstem and spinal cord. Nova proteins harbor three KH-type RNA-binding domains and are expressed exclusively in neurons within the central nervous system (CNS; Buckanovich et al., 1993). KH motifs span about 70 residues with a characteristic pattern of hydrophobic residues, an invariant Gly-X-X-Gly segments, and a variable loop. Nova proteins are closely related to hnRNP E1/E2 (Burd et al., 1994), which regulate α-globin stability (Ostareck-Lederer et al., 1998) and hnRNP K, which shuttles RNA transcripts from the nucleus to the cytoplasm (Michael et al., 1997).

RNA selection experiments demonstrated that Nova proteins are sequence-specific RNA-binding proteins (Buckanovich et al., 1996; Yang et al., 1998). Full-length Nova-1 binds with low-nanomolar affinity to a stem loop RNA. Buckanovich and Darnell (1997) identified a consensus loop sequence [(UCAU(N)₀₋₄]₃ which is necessary and sufficient for binding. They also demonstrated that the stem element confers approximately a three-fold increase in binding affinity but is not essential for binding. This sequence led to the identification of a candidate Nova-1 RNA target in (GlyR α 2) pre-mRNA present in an intron 85 nt up-stream of the inhibitory glycine receptor a2 exon 3A (GlyRα2 E3A; Buckanovich and Darnell, 1997). This exon is alternatively spliced in a mutually exclusive fashion with a downstream exon (E3B; Kuhse et al., 1991). Immunoprecipitation of Nova from brain extracts specifically coprecipitated GlyRα2 pre-mRNA (Buckanovich and Darnell, 1997), providing evidence that a Nova-GlyRα2 protein-RNA complex forms in neurons. Recent X-ray crystallography of a Nova-RNA complex confirms the specificity of the interaction between Nova and the core (UCAUY)₃ motif (Lewis et al., 2000).

### TF111A Protein

TFIIIA regulates the 5S ribosomal RNA genes of *Xenopus laevis* (Sakor et al, 1982; Engelke et al, 1980). This protein binds not only specifically to the promoter DNA, but also to 5S RNA itself (Picard et al, 1979; Pedham et al., 1980; Bogenhagen et al., 1992; Theuissen et al., 1992). The crystal structure of a three-finger complex with 61 bases of RNA, derived from the central regions of the complete nine-finger TFIIIA-5S RNA complex is described in Lu et al., 2003. The structure reveals that the zinc-fingers interact with the backbone of a double helix and that they specifically recognize individual bases positioned for access in otherwise intricately folded 'loop' regions of the RNA.

The locations of the nine fingers have been mapped with respect to the secondary RNA structure, and also their relative contributions to the overall binding affinity have been determined. It has been shown that a 75 nucleotide long RNA, comprising loop A, helix II, helix V, region E and helix IV, bound zinc fingers 4-7 with high affinity. The most important part of the TFIIIA protein for RNA binding is the central set of fingers 4-7, with the major contribution coming from fingers 4-6 and these fingers have been shown to bind 5S-RNA with comparable affinity to the full length protein (Searls et al., 2000; Lu et al, 2003).

### TIS11 Protein

ARE-binding proteins regulate mRNA turnover by promoting or inhibiting degradation, or by influencing translation or subcellular localisation. The TIS11 family of 'immediate early' mRNA-binding proteins are induced in response to direct extracellular stimuli such as insulin, growth factors, mitogens and phorbol esters. They have an important role in mediating the inflammatory response by controlling the activation lifetime of critical genes.

There are three mammalian genes in the TIS11 family - *TIS11d, TIS11b,* and the gene encoding tristetraprolin (*TTP, TIS11, ZFP36,* or *Nup475)* - and a more distantly related gene, XC3H-4, found in *Xenopus laevis* and fish (Hudson et al., 2004). All of the mammalian members of the TIS11 family, TTP, TIS11d and TIS11b, bind to the TNF-α ARE and promote deadenylation and degradation of the mRNA (Lai et al., 2000). TIS11-mediated mRNA degradation seems to occur via the recruitment of the exosome, a multisubunit enzyme complex with 3 - 5 exonuclease activity (Chen et al., 2001).

The mRNA-binding activity of the TIS11 family of proteins resides in a highly conserved TZF domain with two CCCH zinc finger motifs (Fig. 4). The spacing between the cysteine and histidine ligand residues (CX₈CX₅CX₃H) is invariant in the TIS11 proteins, as is the length of the linker between the two zinc finger domains (18 residues) (Fig. 4). In addition, many other residues are strictly conserved, including a (R/K)YKTEL motif immediately preceding the first zinc-coordinating cysteine of each finger. Substitution of many of the conserved amino acids reduces the RNA-binding activity of TTP ZSF domain and leads to an increase in cellular levels of ARE-containing mRNA (Lai et al., 2002).

The TZF domain of TTP is necessary and sufficient for high-affinity binding to its target RNA. A target sequence which has been identified is 5-UUAUUUAUU-3, found within the class II ARE of the 3-UTR of TNF-α mRNA (Lai et al., 1999; Worthington et al., 2002; Blackshear et al., 2003).

### trp RNA-binding attenuation protein (TRAP)

Attenuation is one of several mechanisms regulating the transcription of bacterial operons. This mechanism acts during transcription of the 5' leader regions preceding the structural genes, by folding the nascent RNA into alternative base-paired hairpin structures, one of which signals RNA polymerase to stop transcription. In *Bacillus subtilis* and several other bacilli, the attenuation of the tryptophan biosynthetic genes is controlled by TRAP, which senses the intracellular levels of the amino acid (Antson et al., 1999). TRAP binds a specific RNA sequence in the leader segment of the nascent RNA transcript, but only when activated by bound L-tryptophan (Otridge et al., 1993; Babizke et al., 1993). The RNA target for TRAP contains eleven trinucleotide repeats, almost exclusively made up of GAG or UAG triplets, separated by two or three variable 'spacer' nucleotides (Babitzke et al., 1994; Babitzke et al., 1996). By binding to this RNA, TRAP disrupts or prevents the formation of an 'antiterminator' stem-loop structure, allowing formation of an alternative 'terminator' hairpin that leads to termination of transcription (Gollnick et al., 1994).

When activated by binding L-tryptophan, TRAP binds its cognate RNA with high affinity (K_{d} ≈ 10⁻¹⁰ M; ref. 25). Optimal binding requires that the repeated triplets are separated by two spacer nucleotides Babitzke et al., 1995). Sequence analysis of leader regions from five different bacilli shows 710% GAG, 26% UAG and only one each of AAG and CAG triplets (Chen et al., 1999). The second base is always adenine and the third is always guanine. These two bases are bound to TRAP through a set of intensive interactions (Fig. 4b). Antson et al described a crystal structure of TRAP bound to a 53 base RNA (GAGAU)₁₀GAG (Anston et al, Nature, 1999)

### Pseudouridine Synthase

Pseudouridine (Ψ) is the most abundant modified nucleotide in RNA (Rozenski et al., 1999). In *Escherichia coli,* ten gene products are responsible for the isomerisation of specific uridines in rRNA and tRNA precursors into pseudouridines. The transformation, which involves breakage of the glycosidic bond, rotation of the detached base, and reconnection through C5, does not utilise confactors. By sequence analyses, known Ψ synthases can be grouped into four families, each named for a representative enzyme; RluA, RsuA, TruA, and TruB. Except for members of the TruA family, all other Ψ synthases share short stretches of sequence similarity and are therefore presumed to be descended from a common molecular ancestor (Gustafsson et al., 1996; Koonin, 1996).

The majority of tRNAs in the biosphere carry a Ψ55 residue in their T loop (Dirheimer et al., 1995). TruB family members are responsible for this modification in bacteria, eukaryotes, and probably archaea (Watanabe and Gray, 2000).

RNA hairpins with the sequence of the T stem-loop (TSL) of tRNAs are substrates for the Ψ snythases Pus4 (Becker et al., 1997b) and TruB (Gu et al., 1998). The regioselectivity and kinetics of pseudouridylation are the same on full-length tRNAs and TSL RNAs. Therefore, all determinants of specific Ψ55 synthase-tRNA recognition lie within this segment of tRNAs. (Huang and D'Amare, 2001)Cell, Vol. 107, 929-939,..

### Viral Vector Particle Production Systems

The term 'viral vector particle production system' refers to a system comprising the necessary components for viral particle production.

By using producer/packaging cell lines, it is possible to propagate and isolate quantities of viral vector particles (e.g. to prepare suitable titres of the retroviral vector particles) for subsequent transduction of, for example, a site of interest (such as adult brain tissue). Producer cell lines are usually better for large scale production or vector particles.

As used herein, the term "packaging cell" refers to a cell which contains those elements necessary for production of infectious recombinant virus which are lacking in the RNA genome. Typically, such packaging cells contain one or more producer plasmids which are capable of expressing viral structural proteins (such as codon optimized *gag-pol* and *env)* but they do not contain a packaging signal.

Transient transfection has numerous advantages over the packaging cell method. In this regard, transient transfection avoids the longer time required to generate stable vector-producing cell lines and is used if the vector genome or retroviral packaging components are toxic to cells. If the vector genome encodes toxic genes or genes that interfere with the replication of the host cell, such as inhibitors of the cell cycle or genes that induce apoptosis, it may be difficult to generate stable vector-producing cell lines, but transient transfection can be used to produce the vector before the cells die. Also, cell lines have been developed using transient infection that produce vector titre levels that are comparable to the levels obtained from stable vector-producing cell lines (Pear et al., 1993).

Producer cells/packaging cells can be of any suitable cell type. Producer cells are generally mammalian cells but can be, for example, insect cells.

As used herein, the term "producer cell" or "vector producing cell" refers to a cell which contains all the elements necessary for production of retroviral vector particles.

Preferably, the producer cell is obtainable from a stable producer cell line.

Preferably, the producer cell is obtainable from a derived stable producer cell line.

Preferably the envelope protein sequences, and nucleocapsid sequences are all stably integrated in the producer and/or packaging cell. However, one or more of these sequences could also exist in episomal form and gene expression could occur from the episome.

Also as discussed above, simple packaging cell lines, comprising a provirus in which the packaging signal has been deleted, have been found to lead to the rapid production of undesirable replication competent viruses through recombination. In order to improve safety, second generation cell lines have been produced wherein the 3'LTR of the provirus is deleted. In such cells, two recombinations would be necessary to produce a wild type virus. A further improvement involves the introduction of the *gag-pol* genes and the *env* gene on separate constructs so-called third generation packaging cell lines. These constructs are introduced sequentially to prevent recombination during transfection.

Preferably, the packaging cell lines are second generation packaging cell lines.

Preferably, the packaging cell lines are third generation packaging cell lines.

In these split-construct, third generation cell lines, a further reduction in recombination may be achieved by changing the codons. This technique, based on the redundancy of the genetic code, aims to reduce homology between the separate constructs, for example between the regions of overlap in the *gag-pol* and *env* open reading frames.

The packaging cell lines are useful for providing the gene products necessary to encapsidate and provide a membrane protein for a high titre vector particle production. The packaging cell may be a cell cultured *in vitro* such as a tissue culture cell line. Suitable cell lines include but are not limited to mammalian cells such as murine fibroblast derived cell lines or human cell lines. Preferably the packaging cell line is a human cell line.

Alternatively, the packaging cell may be a cell derived from the individual to be treated. The cell may be isolated from an individual and the packaging and vector components administered *ex vivo* followed by re-administration of the autologous packaging cells.

In more detail, the packaging cell may be an *in vivo* packaging cell in the body of an individual to be treated or it may be a cell cultured *in vitro* such as a tissue culture cell line.

In one embodiment the vector configurations of the present invention use as their production system, three transcription units expressing a genome, the *gag-pol* components and an envelope. The envelope expression cassette may include one of a number of envelopes such as VSV-G or various murine retrovirus envelopes such as 4070A.

### Pseudotyping

In one preferred aspect, the viral vector of the present invention has been pseudotyped. In this regard, pseudotyping can confer one or more advantages. For example, with the lentiviral vectors, the *env* gene product of the HIV-1 based vectors would restrict these vectors to infecting only cells that express a protein called CD4. But if the *env* gene in these vectors has been substituted with *env* sequences from other RNA viruses, then they may have a broader infectious spectrum (Verma and Somia, 1997). By way of example, workers have pseudotyped an HIV-1 based vector with the glycoprotein from VSV (Verma and Somia 1997).

In another alternative, the Env protein may be a modified Env protein such as a mutant or engineered Env protein. Modifications may be made or selected to introduce targeting ability or to reduce toxicity or for another purpose (Valsesia-Wittman *et al* 1996; Nilson *et al* 1996; Fielding *et al* 1998 and references cited therein).

The vector may be pseudotyped with any molecule of choice.

### VSV-G:

The envelope glycoprotein (G) of Vesicular stomatitis virus (VSV), a rhabdovirus, is another envelope protein that has been shown to be capable of pseudotyping certain retroviruses.

Its ability to pseudotype MoMLV-based retroviral vectors in the absence of any retroviral envelope proteins was first shown by Emi et al (1991 Journal of Virology 65:1202-1207). WO94/294440 teaches that retroviral vectors may be successfully pseudotyped with VSV-G. These pseudotyped VSV-G vectors may be used to transduce a wide range of mammalian cells. Even more recently, Abe et al (J Virol 1998 72(8) 6356-6361) teach that non-infectious retroviral particles can be made infectious by the addition of VSV-G.

Burns et al (1993 Proc. Natl. Acad. Sci. USA 90: 8033-7) successfully pseudotyped the retrovirus MLV with VSV-G and this resulted in a vector having an altered host range compared to MLV in its native form. VSV-G pseudotyped vectors have been shown to infect not only mammalian cells, but also cell lines derived from fish, reptiles and insects (Bums *et al* 1993 *ibid*). They have also been shown to be more efficient than traditional amphotropic envelopes for a variety of cell lines (Yee et al, 1994 Proc. Natl. Acad. Sci. USA 91: 9564-9568, Lin, Emi et al, 1991 Journal of Virology 65:1202-1207).

The provision of a non-retroviral pseudotyping envelope such as VSV-G protein gives the advantage that vector particles can be concentrated by ultracentrifugation to a high titre without loss of infectivity (Akkina et al, 1996 J. Virol. 70: 2581-5). Retrovirus envelope proteins are apparently unable to withstand the shearing forces during ultracentrifugation, probably, because they consist of two non-covalently linked subunits. The interaction between the subunits may be disrupted by the centrifugation. In comparison the VSV glycoprotein is composed of a single unit. VSV-G protein pseudotyping can therefore offer potential advantages.

### Ross River Virus

The Ross River viral envelope has been used to pseudotype a nonprimate lentiviral vector (FIV) and following systemic administration predominantly transduced the liver (Kang *et al* 2002). Efficiency was reported to be 20-fold greater than obtained with VSV-G pseudotyped vector, and caused less cytotoxicity as measured by serum levels of liver enzymes suggestive of hepatotoxicity.

Ross River Virus (RRV) is an alphavirus spread by mosquitoes which is endemic and epidemic in tropical and temperate regions of Australia. Antibody rates in normal populations in the temperate coastal zone tend to be low (6% to 15%) although sero-prevalence reaches 27 to 37% in the plains of the Murray Valley River system. In 1979 to 1980 Ross River Virus became epidemic in the Pacific Islands. The disease is not contagious between humans and is never fatal, the first symptom being joint pain with fatigue and lethargy in about half of patients (Fields Virology).

### Baculovirus GP64

The baculovirus GP64 protein has been shown to be an attractive alternative to VSV-G for viral vectors used in the large-scale production of high-titer virus required for clinical and commercial applications (Kumar M, Bradow BP, Zimmerberg J, Hum Gene Ther. 2003 Jan 1;14(1):67-77). Compared with VSV-G, GP64 vectors have a similar broad tropism and similar native titers. Because, GP64 expression does not kill cells, 293T-based cell lines constitutively expressing GP64 can be generated.

### Rabies G

In the present invention the vector system may be pseudotyped with at least a part of a rabies G protein or a mutant, variant, homologue or fragment thereof.

Teachings on the rabies G protein, as well as mutants thereof, may be found in WO 99/61639 and well as Rose et al. 1982 J. Virol. 43: 361-364, Hanham et al. 1993 J. Virol. 67:530-542, Tuffereau et al.1998 J. Virol. 72:1085-1091, Kucera et al. 1985 J. Virol. 55:158-162, Dietzschold et al. 1983 PNAS 80:70-74, Seif et al. 1985 J. Virol. 53:926-934, Coulon et al. 1998 J. Virol. 72:273-278, Tuffereau et a1.1998 J. Virol. 72:1085-10910, Burger et al. 1991 J. Gen. Virol. 72:359-367, Gaudin et al. 1995 J Virol. 69:5528-5534, Benmansour et al. 1991 J. Virol. 65:4198-4203, Luo et al. 1998 Microbiol. Immunol. 42:187-193, Coll 1997 Arch. Virol. 142:2089-2097, Luo et al. 1997 Virus Res. 51:35-41, Luo et al. 1998 Microbiol. Immunol. 42:187-193, Coll 1995 Arch. Virol. 140:827-851, Tuchiya et al. 1992 Virus Res. 25:1-13, Morimoto et al. 1992 Virology 189:203-216, Gaudin et al. 1992 Virology 187:627-632, Whitt et al. 1991 Virology 185:681-688, Dietzschold et al. 1978 J. Gen. Virol. 40:131-139, Dietzschold et al. 1978 Dev. Biol. Stand. 40:45-55, Dietzschold et al. 1977 J. Virol. 23:286-293, and Otvos et al. 1994 Biochim. Biophys. Acta 1224:68-76. A rabies G protein is also described in EP 0445625.

### Alternative envelopes

Other envelopes which give reasonable titre when used to pseudotype EIAV include Mokola, Rabies, Ebola and LCMV (lymphocytic choriomeningitis virus). Following *in utero* injection in mice the VSV-G envelope was found to be more efficient at transducing hepatocytes than either Ebola or Mokola (Mackenzie *et al* 2002). Intravenous infusion into mice of lentivirus pseudotyped with 4070A led to maximal gene expression in the liver (Peng *et al* 2001).

### Delivery Systems

The viral vector of the present invention may be delivered to a target site by a viral or a non-viral vector.

As it is well known in the art, a vector is a tool that allows or facilitates the transfer of an entity from one environment to another. By way of example, some vectors used in recombinant DNA techniques allow entities, such as a segment of DNA (such as a heterologous DNA segment, such as a heterologous cDNA segment), to be transferred into a target cell. Optionally, once within the target cell, the vector may then serve to maintain the heterologous DNA within the cell or may act as a unit of DNA replication. Examples of vectors used in recombinant DNA techniques include plasmids, chromosomes, artificial chromosomes or viruses.

Non-viral delivery systems include but are not limited to DNA transfection methods. Here, transfection includes a process using a non-viral vector to deliver a gene to a target mammalian cell.

Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (Nature Biotechnology 1996 14; 556), and combinations thereof.

Viral delivery systems include but are not limited to adenovirus vector, an adeno-associated viral (AAV) vector, a herpes viral vector, retroviral vector, lentiviral vector, baculoviral vector. Other examples of vectors include *ex vivo* delivery systems, which include but are not limited to DNA transfection methods such as electroporation, DNA biolistics, lipid-mediated transfection, compacted DNA-mediated transfection.

The vector delivery system of the present invention may consist of a primary vector manufactured *in vitro* which encodes the genes necessary to produce a secondary vector *in vivo.*

The primary viral vector or vectors may be a variety of different viral vectors, such as retroviral, adenoviral, herpes virus or pox virus vectors, or in the case of multiple primary viral vectors, they may be a mixture of vectors of different viral origin. In whichever case, the primary viral vectors are preferably defective in that they are incapable of independent replication. Thus, they are capable of entering a target cell and delivering the secondary vector sequences, but not of replicating so as to go on to infect further target cells.

### Nucleotide Sequence of Interest (NOI)

The viral vector of the present invention may be used to deliver one or more NOI(s) useful in the treatment of the disorders listed in WO-A-98/05635. The nucleotide sequence of interest may be DNA or RNA. For ease of reference, part of that list is now provided: cancer, inflammation or inflammatory disease, dermatological disorders, fever, cardiovascular effects, haemorrhage, coagulation and acute phase response, cachexia, anorexia, acute infection, HIV infection, shock states, graft-versus-host reactions, autoimmune disease, reperfusion injury, meningitis, migraine and aspirin-dependent anti-thrombosis; tumour growth, invasion and spread, angiogenesis, metastases, malignant, ascites and malignant pleural effusion; cerebral ischaemia, ischaemic heart disease, osteoarthritis, rheumatoid arthritis, osteoporosis, asthma, multiple sclerosis, neurodegeneration, Alzheimer's disease, atherosclerosis, stroke, vasculitis, Crohn's disease and ulcerative colitis; periodontitis, gingivitis; psoriasis, atopic dermatitis, chronic ulcers, epidermolysis bullosa; corneal ulceration, retinopathy and surgical wound healing; rhinitis, allergic conjunctivitis, eczema, anaphylaxis; restenosis, congestive heart failure, endometriosis, atherosclerosis or endosclerosis.

In addition, or in the alternative, the viral vector of the present invention may be used to deliver one or more NOI(s) useful in the treatment of disorders listed in WO-A-98/07859. For ease of reference, part of that list is now provided: cytokine and cell proliferation/differentiation activity; immunosuppressant or immunostimulant activity (e.g. for treating immune deficiency, including infection with human immune deficiency virus; regulation of lymphocyte growth; treating cancer and many autoimmune diseases, and to prevent transplant rejection or induce tumour immunity); regulation of haematopoiesis, e.g. treatment of myeloid or lymphoid diseases; promoting growth of bone, cartilage, tendon, ligament and nerve tissue, e.g. for healing wounds, treatment of bums, ulcers and periodontal disease and neurodegeneration; inhibition or activation of follicle-stimulating hormone (modulation of fertility); chemotactic/chemokinetic activity (e.g. for mobilizing specific cell types to sites of injury or infection); haemostatic and thrombolytic activity (e.g. for treating haemophilia and stroke); antiinflammatory activity (for treating e.g. septic shock or Crohn's disease); as antimicrobials; modulators of e.g. metabolism or behaviour; as analgesics; treating specific deficiency disorders; in treatment of e.g. psoriasis, in human or veterinary medicine.

In addition, or in the alternative, the viral vector of the present invention may be used to deliver one or more NOI(s) useful in the treatment of disorders listed in WO-A-98/09985. For ease of reference, part of that list is now provided: macrophage inhibitory and/or T cell inhibitory activity and thus, anti-inflammatory activity; anti-immune activity, i.e. inhibitory effects against a cellular and/or humoral immune response, including a response not associated with inflammation; inhibit the ability of macrophages and T cells to adhere to extracellular matrix components and fibronectin, as well as up-regulated fas receptor expression in T cells; inhibit unwanted immune reaction and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery, bone marrow transplantation or other transplantation complications and/or side effects, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

### Therapeutic RNA

By therapeutic RNA is meant a sequence which functions at the RNA level. Preferably the therapeutic RNA does not require integration to have a therapeutic effect. More preferably the therapeutic RNA does not require reverse transcription to have a therapeutic effect.

Examples of such RNA include siRNA,shRNA, micro-RNA,or regulated sh or micro RNA (Dickins et al, 2005, Nature Genetics 37: 1289-1295; Silva et al 2005 Nature Genetics 37: 1281-1288) a ribozyme, an mRNA or a tRNA. The vector particle may also be used to deliver an antisense sequence.

Post-transcriptional gene silencing (PTGS) mediated by double-stranded RNA (dsRNA) is a conserved cellular defense mechanism for controlling the expression of foreign genes. It is thought that the random integration of elements such as transposons or viruses causes the expression of dsRNA which activates sequence-specific degradation of homologous single-stranded mRNA or viral genomic RNA. The silencing effect is known as RNA interference (RNAi) (Ralph et al, 2005, Nature Medicine 11: 429-433). The mechanism of RNAi involves the processing of long dsRNAs into duplexes of 21-25 nucleotide (nt) RNAs. These products are called small interfering or silencing RNAs (siRNAs) which are the sequence-specific mediators of mRNA degradation. In differentiated mammalian cells dsRNA >30bp has been found to activate the interferon response leading to shut-down of protein synthesis and non-specific mRNA degradation (Stark et al 1998). However this response can be bypassed by using 21nt siRNA duplexes (Elbashir et al 2001, Hutvagner et al 2001) allowing gene function to be analysed in cultured mammalian cells.

In another embodiment the NOI comprises a micro-RNA. Micro-RNAs are a very large group of small RNAs produced naturally in organisms, at least some of which regulate the expression of target genes. Founding members of the micro-RNA family are *let-7* and *lin-4.* The *let-7* gene encodes a small, highly conserved RNA species that regulates the expression of endogenous protein-coding genes during worm development. The active RNA species is transcribed initially as an ∼70nt precursor, which is post- transcriptionally processed into a mature ∼21nt form. Both *let-7* and *lin-4* are transcribed as hairpin RNA precursors which are processed to their mature forms by Dicer enzyme.

### Transient Expression

It may be desirable, in a therapeutic setting, to be able to transiently express proteins or transiently knock-down expression of proteins. This may be achieved by delivering RNA to target cells. RNA has a finite lifetime in cells and once it has been degraded the phenotype it conferred on cells would be removed. This has been difficult to achieve to date because of a lack of suitable vectors and transfection methods for RNA have yet to achieve satisfactory levels of transfer *in vivo.*

The retroviral constructs of the present invention can be used to specifically package therapeutic RNA which is free of retroviral RNA, for efficient delivery *in vivo.* This is achieved by incorporating into gag or gag/pol a heterologous RNA binding domain. This RNA binding domain binds a corresponding RNA sequence inserted into the RNA entity that is to be delivered to the target cells.

For transient expression, it is preferable if the retroviral vector is integration deficient and/or unable to undergo reverse transcription.

A preferred NOI of interest used for transient expression is an siRNA or micro-RNA.

### Promoters

Expression of a NOI may be controlled using control sequences, which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters.

Suitable promoting sequences are strong promoters including those derived from the genomes of viruses - such as polyoma virus, adenovirus, fowlpox virus, bovine papilloma virus, avian sarcoma virus, cytomegalovirus (CMV), retrovirus and Simian Virus 40 (SV40) - or from heterologous mammalian promoters - such as the actin promoter or ribosomal protein promoter. Transcription of a gene may be increased further by inserting an enhancer sequence into the vector. Enhancers are relatively orientation and position independent, however, one may employ an enhancer from a eukaryotic cell virus - such as the SV40 enhancer on the late side of the replication origin (bp 100-270) and the CMV early promoter enhancer. The enhancer may be spliced into the vector at a position 5' or 3' to the promoter, but is preferably located at a site 5' from the promoter.

The promoter can additionally include features to ensure or to increase expression in a suitable host. For example, the features can be conserved regions e.g. a Pribnow Box or a TATA box. The promoter may even contain other sequences to affect (such as to maintain, enhance, decrease) the levels of expression of a nucleotide sequence. Suitable other sequences include the Sh1-intron or an ADH intron. Other sequences include inducible elements - such as temperature, chemical, light or stress inducible elements. Also, suitable elements to enhance transcription or translation may be present.

### Pharmaceutical Compositions

We also describe herein a pharmaceutical composition for treating an individual by gene therapy, wherein the composition comprises a therapeutically effective amount of the viral vector of the present invention comprising one or more deliverable therapeutic and/or diagnostic NOI(s) or a viral particle produced by or obtained from same. The pharmaceutical composition may be for human or animal usage. Typically, a physician will determine the actual dosage which will be most suitable for an individual subject and it will vary with the age, weight and response of the particular individual.

The composition may optionally comprise a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system).

Where appropriate, the pharmaceutical compositions can be administered by any one or more of: inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavemosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### Treatment

It is to be appreciated that all references herein to treatment include curative, palliative and prophylactic treatment. The treatment of mammals is particularly preferred. Both human and veterinary treatments are within the scope of the present invention.

Various preferred features and embodiments of the invention will now be described by way of non-limiting examples with reference to the accompanying Examples.

### Example 1

We describe how the MS2 recognition sequence and binding protein may be used to replace the existing packaging signal/gag-pol interaction in an EIAV vector system. The principle may be used for any vector system where there is packaging of RNA molecules into viral particles through RNA binding proteins.

The starting plasmid could be pONY8.4NCG or pONY8.9NCG (described in WO 03/064665). The RNA sequence that is essential for packaging and therefore is considered to be the packaging signal has previously been described (Mitrophanous et al (1999) Gene Ther. 6(11):1808-18). In one embodiment this sequence would be excised and replaced with the target RNA sequence (e.g. from MS2) using standard molecular biology techniques. Alternatively, the MS2 sequence could be added between the primer binding site and the polypurine tract. In conjunction, the gag-pol protein expression plasmid, pESYNGP (described in WO 01/79518), would be modified. This construct could have the sequence found in the nucleocapsid protein which codes for the packaging recognition amino acids removed and replaced with the sequence that results in the expression of the RNA binding domain from the MS2 coat protein; this may be achieved using standard molecular biology techniques. An alternative would be to insert the MS2 coat protein within or at the end of gag or gag/pol. The MS2 coat protein could also be inserted within the dUTPase domain of the pol protein

A series of constructs may be made to find the optimal location of the RNA motif within the RNA genome and the optimal location of the MS2 coat protein within the gag-pol.

The vector system could be tested with a standard co-transfection to make vector and titred in the standard manner.

The aim of the following experiments was to determine the ability of the EIAV packaging signal to package non-native (ie non-EIAV) RNA into vector particles by determining the RNA copy number per ml following vector production. The non-native RNA sequence was capable of expressing GFP. The degree of GFP expression was also measured.

The construction of two plasmids is described below (pONY8.0NCG and pPSIEGFP-N1). pONY8.0NCG contained the neo ORF and was obtained by excising the neo ORF from pONY8.9NCG (desribed in WO 03/064665) and ligating it into pONYB.0G through digestion with BgIII and BsmBI.

The plasmid pONYB.0NCG was designed to test whether the insertion of the upstream open reading frame, in this instance Neo, confers REV independence in the presence of the RRE and instability sequences located in the deleted envelope region of this plasmid. The vector genome pONY8.9NCG is REV-independent and lacks the envelope regions. The vectors produced from transfections with or without REV were tested for their biological titre, RNA copy number per ml and RT activity (via performance enhanced reverse transcriptase assay -PERT) of the viral particle.

The plasmid pPSIEGFP-N1 was obtained by inserting the EIAV packaging signal (Ψ) from pONY8.4GCZ into the commercially available GFP-expressing plasmid, pEGFP-N1 (Clontech), by digesting with SnaBI and Sall. The plasmids pPSIEGFP-N1 and pEGFP-N1 were transfected along with the packaging components of the EIAV vector system (gag/pol and VSV-G) to determine whether the inclusion of the packaging signal into pPSIEGFP-N1 was sufficient to confer efficient RNA packaging.

Vector preparations were made by transient co-transfection of 293T human embryonic kidney cells with the vectors and additional plasmids as detailed in Table 1 whereby all transfections contain Gag/Pol (pESYNGP) and envelope pRV67 (VSV-G). After vector harvest transfected cells were analysed by FACS. The cells were centrifuged and the pellets stored at -80°C. Cell culture supernatants were tested by titring on D17 cells, by PERT assay and RNA copy number per ml assay.

The PERT assay has been previously described by Arnold et al (1998) Biotechniques 25; 98-106. The RNA copy number per ml assay was performed as described in Rohll et al (2002) Methods in Enzymology 346: 466-500. The biological titre (GFP reporter gene expression in target cells) was assessed by FACS analysis using a method similar to that described in Siapati et al (2005) Molecular Therapy 12; 537-546.

The results of these tests are shown in Table 1

The RNA copy number per ml results show that the RNA transcript from pPSIEGFP-N1 was packaged as efficiently as those from the EIAV genomes (eg pONYB.0NCG). Table 1 shows that insertion of neo as described in pONYB.0NCG confers Rev-independence (compare ID 4 of Table 1 with IDs 7&8). It should be noted that RNA copy number per ml and RT activity (PERT) were similar showing that Rev is not required for efficient packaging and particle production of pONY8.0NCG.

**Table 1. Summary table of results**

| Plate ID | Genome | Extra plasmid | Transfection % | Biological titre (TU/ml) | PERT predicted titre (TU/ml) | Ψ RNA copy number per ml |
|---|---|---|---|---|---|---|
| 1 | pONY8.1G | None | 62 | - | 3.97E+06 | 2.86e8 |
| 2 | pONY8.1G | pCINeo | 65 | - | 1.89E+06 | 1.15e8 |
| 3 | pONYB.1G | pESYNREV | 62 | 1.5e+04 | 1.67E+06 | 2.29e9 |
| 4 | pONYB.0G | pCINeo | 49 | ND | 2.25E+06 | 4.21e7 |
| 5 | pONY8.4ZCG | pCINeo | 68 | 6.4e+4 | 2.87E+06 | 1.07e10 |
| 6 | pONY8.4ZCG | pESYNREV | 52 | 1.6e+5 | 2.88E+06 | 2.03e10 |
| 7 | pONY8.0NCG | pCINeo | 51 | 4.6e+5 | 2.78E+06 | 1.25e10 |
| 8 | pONY8.0NCG | pESYNREV | 57 | 1.4e+6 | 3.26E+06 | 1.36e10 |
| 9 | Blank | pCINeo | 0 | - | 1.49E+06 | |
| 10 | Blank | pESYNREV | 0 | - | 1.18E+06 | |
| 11 | Blank | None | 0 | - | 1.93E+06 | |
| 12 | pEGFP-N1 | None | 71 | - | 3.48E+05 | ND |
| 13 | pPSIEGFP-N1 | None | 53 | - | 1.82E+06 | 2.76e10 |
| | Int - | | | 3.47e+4 | 3.12E+06 | 1.41e10 |
| | Int+ | | | 8.09e+5 | 3.66E+06 | 1.14e10 |

It has thus been shown that a GFP-expressing plasmid (pPSIEGFP-N1) could transcribe RNA that could be packaged into EIAV particles when the RNA included the EIAV packaging signal (Ψ). The level of packaging was at least as high as the level of packaging of other EIAV vector genomes which were prepared alongside.

As the packaging signal is absent from pEGFP-N1 an accurate RNA copy number cannot be measured. However, both pEGFP-N1 and pPSIEGFP-N1 contain GFP sequence. Therefore RNA copy number was measured using a TaqMan assay to measure RNA containing the GFP sequence. For pEGFP-N1 and pPSIEGFP-N1 1.3x10⁷ and 3.7x10⁹ copies per ml were detected, respectively. This shows that the presence of the packaging sequence increased packaging of the non-native RNA by 280-fold.

### References

Lewis et al., EMBO. J 11:3053-3056 (1992)
Lewis and Emerman, K. Virol. 68:510-516 (1994)
Derse and Newbold, Virology 194:530-6 (1993)
Maury et al., Virology 200:632-42 (1994)
Martarano et al., J Virol. 68:3102-11 (1994)
Bender et al., J. Virol., 61 : 1639-1646 (1987)
Kaye et al., J Virol. Oct;69(10):6588-92 (1995)
Grahn et al., RNA, 7:1616-1627 (2001)
Witherell et al, Progr Nucleic Acid Res Mol Biol 40:185-220 (1991)
Lowary et al., Nucleic Acids Res 15:10483-10493 (1987)
Talbot et al., Nucleic Acids Res 18:3521-3528 (1990)
Lago et al., Nucleic Acids Res 26:1337-1344 (1998)
Carey et al., Biochemistry 22:4723-4730 (1983a)
Battiste et al., Science, Vol. 273, 1547-1548 (1996)
Olsen et al., Genes Dev. 4, 1357 (1990)
Zapp et al., Proc. Natl Acad Sci. USA 88, 7734 (1991)
Tan et al., Cell 73, 1031 (1993)
Lührmann et al., biophys. Acta 1087, 265-292 (1990)
Nagai et al., in RNA-protein interactions, 150-176 (IRL, Oxford, 1994)
Sillekens et al, EMBO J. 6, 3841-3848 (1987)
Burd et al., Science, 265, 615-621 (1994)
Birney et al., Nucleic Acids Res. 21, 5803-5816 (1993)
Scherley et al., EMBO J. 8, 4163-4170 (1989)
Bach et al., Nucleic Acids Res. 18, 449-457 (1990)
Scherley et al., Nature 345, 502-506 (1990)
Hall, K. B., Biochemistry 33, 10076-10088 (1994)
Nagai et al, Nature 348, 515-520 (1990)
Jensen et al., EMBO J. 10, 3447-3456 (1991)
Boelens et al., Cell 72, 881-892, (1993)
Lu et al., Nature, Vol. 426, November 2003
Sakor et al, Cell 31, 395-405 (1982)
Engelke et al, Cell 19, 717-728 (1980)
Picard et al, Proc. Natl Acad. Sci. USA 76, 241-245 (1979)
Pedham et al., Proc. Natl. Acad. Sci. USA 77, 4170-4174 (1980)
Bogenhagen et al., Nucleic Acids Res. 20, 2639-2645 (1992)
Theuissen et al., Cell, 71, 679-690 (1992)
Searls et al., J. Mol. Bio. 301, 47-60 (2000).
Hudson et al., Nature Structural & Molecule Biological, Vol 11, 3, 257 & 262 (2004)
Lai et al., J. Biol. Chem. 275, 17827-17837 (2000)
Chen et al., Cell 107, 451-464 (2001)
Lai et al., J. Biol. Chem. 277, 9606-9613 (2002)
Worthington et al., J. Biol. Chem. 277, 48558-48564 (2002)
Blackshear et al., J. Biol. Chem. 278, 19947 (2003)
Antson et al., Nature, Vol. 401, 235 & 238 (1999)
Otridge et al., Proc. Natl. Acad. Sci. USA 90, 128-132 (1993)
Babitzke et al., Proc. Natl. Acad. Sci. USA 90, 133-137 (1993)
Babitzke et al., J. Biol. Chem. 269, 16597-16604 (1994)
Babitzke et al., J. Bacteriol. 178, 5159-5163 (1996)
Gollnick, P., Mol. Microbiol. 11, 991-997 (1994)
Babitzke et al., Proc. Natl. Acad. Sci. USA 92, 7916-7920 (1995)
Chen et al., J. Mol. Biol. 289, 1003-1016 (1999)
Gustafsson et al., Nucleic Acids Res. 24, 3756-3762 (1996)
Koonin, Nucleic Acids Res. 24, 2411-2415 (1996)
Dirheimer et al., 1995, In tRNA: Structure, Biosynthesis and Function, D Soll and U. RajBhandary eds. (Washington, D.C.: American Society for Microbiology Press) pp 93-126
Watanabe and Gray, Nucleic Acids Res. 28, 2342-2352 (2000)
Becker et al., J. Mol. Biol. 274, 505-518 (1997)
Gu et al., Biochemistry 37, 339-343 (1998)
Lewis et al., Cell, Vol 100, 323-332, (2000)
Baber et al., J. Mol. Biol. 289, 949-962 (1999)
Buckanovich and Darnell Mol. Cell. Biol. 17, 3194-3201 (1997)
Buckanovich et al., Neuron 11, 657-672 (1993)
Ostareck-Lederer et al., Trends Biochem. Sci. 23, 409-411 (1998)
Michael et al., EMBO J. 16, 3587-3598 (1997)
Yang et al., Proc. Natl. Acad. Sci. USA 95, 13254-13259 (1998)
Kuhse et al., FEBS Lett. 283, 73-77 (1991)
Pear er al.m PNAS 90:8392-8396 (1993)
Verma and Somia, Nature 389-239-424 (1997)
Valsesia-Wittman et al 1996 J Virol 70:2056-64
Nilson et al., Gene Therapy 3 : 280-6 (1996)
Fielding et al., Blood 9: 1802 (1998)
Stark et al., Annu Rev Biocheh 67:227-64 (1998)
Elbashir et al., EMBO J. Dec 3;20(23):6877-88 (2001)
Hutvagner et al., Science.Aug 3, 293(5531):834-8. Eupub Jul 12 (2001)

## Claims

1. A retroviral vector particle production system comprising
Env;
retroviral Gag, or retroviral Gag/Pol, containing a binding domain;
and an RNA sequence to be packaged containing a retroviral packaging signal or a corresponding cognate packaging signal which is recognized by said binding domain to facilitate packaging of the RNA sequence into a retroviral vector particle, wherein said RNA sequence to be packaged lacks any other retroviral sequences including viral sequences required for reverse transcription and integration.

2. The retroviral vector particle production system of claim 1 wherein the binding domain is endogenous to retroviral Gag or retroviral Gag/Pol, the RNA sequence to be packaged contains the retroviral packaging signal recognized by said binding domain, and said RNA sequence to be packaged lacks any other retroviral sequences.

3. The retroviral vector particle production system of claim 1 wherein the binding domain is heterologous to retroviral Gag or retroviral Gag/Pol.

4. The retroviral vector particle production system of claim 3 wherein the heterologous binding domain is retroviral, the RNA sequence to be packaged contains the retroviral packaging signal recognized by said binding domain, and said RNA sequence to be packaged lacks any other retroviral sequences.

5. The retroviral vector particle production system of claim 3 wherein the binding domain is non-viral, the RNA sequence to be packaged contains the non-viral packaging signal recognized by said binding domain, and said RNA sequence to be packaged lacks all retroviral sequences.

6. The retroviral vector particle production system of claim 1 wherein the heterologous binding domain contains a binding domain derived from a bacteriophage coat protein, a Rev protein, a protein of the U1 small nuclear ribonucleoprotein particle, a Nova protein, a TF111A protein, a TIS11 protein, a trp RNA-binding attenuation protein (TRAP) or a pseudouridine synthase.

7. A retroviral vector particle comprising Env and retroviral Gag or retroviral Gag/Pol, wherein said retroviral Gag and retroviral Gag/Pol comprises a RNA binding domain, and wherein said retroviral vector particle further comprises a packaged RNA sequence containing a retroviral packaging signal or a corresponding cognate packaging signal which is recognized by said binding domain to facilitate packaging of the RNA sequence into a viral vector particle, wherein said packaged RNA sequence lacks any other retroviral sequences including retroviral sequences required for reverse transcription and integration.

8. A retroviral vector particle production system or a retroviral vector particle according to any of the preceding claims wherein Gag or Gag/Pol is derived from a lentivirus.

9. A retroviral vector particle production system or a retroviral vector particle according to claim 8 wherein Gag or Gag/Pol is derived from HIV or EIAV.

10. A retroviral vector particle production system or a retroviral vector particle according to any of the preceding claims wherein the RNA sequence further comprises a nucleotide sequence of interest (NOI).

11. A retroviral vector particle production system or a retroviral vector particle according to claim 10 wherein the NOI is a therapeutic RNA.

12. A retroviral vector particle production system or a retroviral vector particle according to claim 11 wherein the therapeutic RNA is selected from the group of siRNA, micro-RNA, ribozyme, mRNA and tRNA.

## Patentansprüche

1. Retrovirus-Vektorpartikel-Produktionssystem, umfassend
Env;
retrovirales Gag, oder retrovirales Gag/Pol, mit einer Bindungsdomäne;
und eine zu verpackende RNA-Sequenz mit einem retroviralen Verpackungssignal oder einem entsprechenden zugehörigen Verpackungssignal, das von der Bindungsdomäne erkannt wird, so dass die Verpackung der RNA-Sequenz in ein Retrovirus-Vektorpartikel erleichtert wird, wobei der zu verpackenden RNA-Sequenz alle anderen retroviralen Sequenzen, einschließlich für reverse Transkription und Integration benötigte virale Sequenzen, fehlen.

2. Retrovirus-Vektorpartikel-Produktionssystem nach Anspruch 1, wobei die Bindungsdomäne endogen für retrovirales Gag oder retrovirales Gag/Pol ist, die zu verpackende RNA-Sequenz das von der Bindungsdomäne erkannte retrovirale Verpackungssignal enthält und der zu verpackenden RNA-Sequenz alle anderen retroviralen Sequenzen fehlen.

3. Retrovirus-Vektorpartikel-Produktionssystem nach Anspruch 1, wobei die Bindungsdomäne heterolog zu retroviralem Gag oder retroviralem Gag/Pol ist.

4. Retrovirus-Vektorpartikel-Produktionssystem nach Anspruch 3, wobei die heterologe Bindungsdomäne retroviral ist, die zu verpackende RNA-Sequenz das von der Bindungsdomäne erkannte retrovirale Verpackungssignal enthält und der zu verpackenden RNA-Sequenz alle anderen retroviralen Sequenzen fehlen.

5. Retrovirus-Vektorpartikel-Produktionssystem nach Anspruch 3, wobei die Bindungsdomäne nicht viral ist, die zu verpackende RNA-Sequenz das von der Bindungsdomäne erkannte nicht virale Verpackungssignal enthält und der zu verpackenden RNA-Sequenz alle retroviralen Sequenzen fehlen.

6. Retrovirus-Vektorpartikel-Produktionssystem nach Anspruch 1, wobei die heterologe Bindungsdomäne eine von einem Bakteriophagen-Hüllprotein, einem Rev-Protein, einem Protein des kleinen nukleären U1-Ribonukleoprotein-Partikels, einem Nova-Protein, einem TF111A-Protein, einem TIS11-Protein, einem TRAP (trp RNA-binding attenuation protein) oder einer Pseudouridin-Synthase abgeleitete Bindungsdomäne enthält.

7. Retrovirus-Vektorpartikel, umfassend Env und retrovirales Gag oder retrovirales Gag/Pol, wobei das retrovirale Gag bzw. retrovirale Gag/Pol eine RNA-Bindungsdomäne umfasst und wobei das Retrovirus-Vektorpartikel ferner eine verpackte RNA-Sequenz mit einem retroviralen Verpackungssignal oder einem entsprechenden zugehörigen Verpackungssignal, das von der Bindungsdomäne erkannt wird, so dass die Verpackung der RNA-Sequenz in ein Virus-Vektorpartikel erleichtert wird, wobei der verpackten RNA-Sequenz alle anderen retroviralen Sequenzen, einschließlich für reverse Transkription und Integration benötigte retrovirale Sequenzen, fehlen.

8. Retrovirus-Vektorpartikel-Produktionssystem oder Retrovirus-Vektorpartikel gemäß einem der vorhergehenden Ansprüche, wobei Gag oder Gag/Pol aus einem Lentivirus stammt.

9. Retrovirus-Vektorpartikel-Produktionssystem oder Retrovirus-Vektorpartikel gemäß Anspruch 8, wobei Gag oder Gag/Pol aus HIV oder EIAV stammt.

10. Retrovirus-Vektorpartikel-Produktionssystem oder Retrovirus-Vektorpartikel gemäß einem der vorhergehenden Ansprüche, wobei die RNA-Sequenz ferner eine interessierende Nukleotidsequenz (NOI) umfasst.

11. Retrovirus-Vektorpartikel-Produktionssystem oder Retrovirus-Vektorpartikel gemäß Anspruch 10, wobei es sich bei der NOI um eine therapeutische RNA handelt.

12. Retrovirus-Vektorpartikel-Produktionssystem oder Retrovirus-Vektorpartikel gemäß Anspruch 11, wobei die therapeutische RNA aus der Gruppe siRNA, micro-RNA, Ribozym, mRNA und tRNA ausgewählt ist.

## Revendications

1. Système de production de particules d'un vecteur rétroviral comprenant
un Env ;
un Gag rétroviral ou un Gag/Pol rétroviral, contenant un domaine de liaison ;
ainsi qu'une séquence d'ARN devant être encapsidée, contenant un signal d'encapsidation rétroviral ou un signal d'encapsidation cognat correspondant qui est reconnu par ledit domaine de liaison pour faciliter l'encapsidation de la séquence d'ARN dans une particule d'un vecteur rétroviral, dans lequel ladite séquence d'ARN devant être encapsidée est dépourvue d'une quelconque autre séquence rétrovirale, y compris des séquences virales requises pour une transcription inverse et une intégration.

2. Système de production de particules d'un vecteur rétroviral selon la revendication 1, dans lequel le domaine de liaison est endogène à un Gag rétroviral ou un Gag/Pol rétroviral, la séquence d'ARN devant être encapsidée contient le signal d'encapsidation rétroviral reconnu par ledit domaine de liaison, et ladite séquence d'ARN devant être encapsidée est dépourvue d'une quelconque autre séquence rétrovirale.

3. Système de production de particules d'un vecteur rétroviral selon la revendication 1, dans lequel le domaine de liaison est hétérologue à un Gag rétroviral ou un Gag/Pol rétroviral.

4. Système de production de particules d'un vecteur rétroviral selon la revendication 3, dans lequel le domaine hétérologue de liaison est rétroviral, la séquence d'ARN devant être encapsidée contient le signal d'encapsidation rétroviral reconnu par ledit domaine de liaison, et ladite séquence d'ARN devant être encapsidée est dépourvue d'une quelconque autre séquence rétrovirale.

5. Système de production de particules d'un vecteur rétroviral selon la revendication 3, dans lequel le domaine de liaison est non viral, la séquence d'ARN devant être encapsidée contient le signal d'encapsidation non viral reconnu par ledit domaine de liaison, et ladite séquence d'ARN devant être encapsidée est dépourvue de toute séquence rétrovirale.

6. Système de production de particules d'un vecteur rétroviral selon la revendication 1, dans lequel le domaine hétérologue de liaison contient un domaine de liaison dérivé d'une protéine de l'enveloppe d'un bactériophage, d'une protéine Rev, d'une protéine de la petite particule ribonucléoprotéique nucléaire U1, d'une protéine Nova, d'une protéine TF111A, d'une protéine TIS11, d'une protéine trp d'atténuation de la liaison à l'ARN (TRAP) ou d'une pseudo-uridine synthase.

7. Particule d'un vecteur rétroviral comprenant un Env et un Gag rétroviral ou un Gag/Pol rétroviral, dans laquelle lesdits Gag rétroviral et Gag/Pol rétroviral comprennent un domaine de liaison à l'ARN et dans laquelle ladite particule d'un vecteur rétroviral comprend en outre une séquence d'ARN encapsidée contenant un signal d'encapsidation rétroviral ou un signal d'encapsidation cognat correspondant qui est reconnu par ledit domaine de liaison pour faciliter l'encapsidation de la séquence d'ARN dans une particule d'un vecteur viral, dans laquelle ladite séquence d'ARN encapsidée est dépourvue d'une quelconque autre séquence rétrovirale, y compris des séquences rétrovirales requises pour une transcription inverse et une intégration.

8. Système de production de particules d'un vecteur rétroviral ou particule d'un vecteur rétroviral selon l'une quelconque des revendications précédentes, dans lesquels Gag ou Gag/Pol est dérivé d'un lentivirus.

9. Système de production de particules d'un vecteur rétroviral ou particule d'un vecteur rétroviral selon la revendication 8, dans lesquels Gag ou Gag/Pol est dérivé d'un VIH ou d'un VAIE.

10. Système de production de particules d'un vecteur rétroviral ou particule d'un vecteur rétroviral selon l'une quelconque des revendications précédentes, dans lesquels la séquence d'ARN comprend en outre une séquence nucléotidique d'intérêt (NOI).

11. Système de production de particules d'un vecteur rétroviral ou particule d'un vecteur rétroviral selon la revendication 10, dans lesquels la NOI est un ARN thérapeutique.

12. Système de production de particules d'un vecteur rétroviral ou particule d'un vecteur rétroviral selon la revendication 11, dans lesquels l'ARN thérapeutique est choisi dans le groupe des ARNsi, micro-ARN, ribozyme, ARNm et ARNt.
